# EUROPÄISCHE PATENTSCHRIFT

(11) **EP 1 516 604 B1**
(45) Veröffentlichungstag und Bekanntmachung des Hinweises auf die Patenterteilung: **21.05.2008**
(21) Anmeldenummer: 04017730.5
(22) Anmeldetag: 27.07.2004
(51) Int. Cl.: A61F 5/56

(54) **Intraorales Therapiegerät**
Intra-oral therapeutic device
Dispositif thérapeutique intra-oral

(30) Priorität: 16.09.2003 DE 10343051; 08.12.2003 DE 20319088 U
(43) Veröffentlichungstag der Anmeldung: 23.03.2005
(73) Patentinhaber: Dr. Hinz Labor, Fachlaboratorium für Kieferorthopädie GmbH & Co. KG, D-44623 Herne (DE)
(72) Erfinder: Hinz, Rolf, Prof.Dr., 44623 Herne (DE)
(74) Vertreter: Gesthuysen, von Rohr & Eggert

(56) Entgegenhaltungen:
- DE-C- 10 216 242
- US-A- 5 499 633
- US-A- 6 055 986
- US-B1- 6 418 933

## Beschreibung

Die Erfindung betrifft ein intraorales Therapiegerät nach dem Oberbegriff des Anspruchs 1.

Intraorale Therapiegeräte zur Schnarchtherapierung der eingangs genannten Art sind bereits aus der DE - C - 102 16 242 und DE - U - 295 06 512 bekannt. Nachteilig bei den bekannten Schnarch-Therapiegeräten ist, daß der Patient die seitlich angeordneten Stäbe als störend empfindet. So kann es an den Wangeninnenseiten im Bereich der Stäbe, insbesondere im Bereich der Anlenkpunkte der Stäbe, zu unangenehmen Druckstellen kommen. Durch eine vermehrte Bewegung des Unterkiefers während des Tragens des Schnarch-Therapiegerätes kann es zudem zu Reizungen, im schlimmsten Fall zu Entzündungen des Gewebes an der Innenseite der Wangen kommen. Darüber hinaus kommt es zu einer steten Berührung zwischen Wangen und Stäben, wenn das Gerät getragen wird. Aus diesem Grunde hat der Patient stets das unangenehme Gefühl, als ob er einen Fremdkörper im Mund tragen würde. Die bekannten Geräte erfahren daher nur eine geringe Akzeptanz.

Ein Therapiegerät gemäß den Merkmalen des Oberbegriffs des Anspruchs 1 ist aus der DE-C-10216242 bekannt.

Aufgabe der vorliegenden Erfindung ist es daher, ein intraorales Therapiegerät der eingangs genannten Art zur Verfügung zu stellen, das einen höheren Tragekomfort bietet und eine bessere Akzeptanz seitens der Benutzer erfährt.

Zur Lösung der vorgenannten Aufgabe dienen bei dem intraoralen Therapiegerät der eingangs genannten Art die Merkmale des Anspruchs 1.

Daß der Stab interokklusal, also zwischen den Bißflächen, angeordnet ist und einerseits an einem seitlichen, oberen Aufbiß der Oberkieferschiene, d.h. der diesbezüglichen oberen Aufbißfläche, und andererseits an dem dem oberen Aufbiß gegenüberliegenden, unteren seitlichen Aufbiß der Unterkieferschiene, d.h. der diesbezüglichen unteren Aufbißfläche, gelenkig gelagert ist, ist eine vorteilhafte Ausgestaltung der Erfindung. Der Stab befindet sich also zwischen den beiden Schienen, und zwar auch bezüglich seiner Lagerung.

Obwohl es in diesem Zusammenhang grundsätzlich möglich ist, lediglich einen Stab als Verbindung zwischen der Oberkieferschiene und der Unterkieferschiene vorzusehen, bietet es sich zur besseren Führung an, die beiden Schienen über zwei Führungsstäbe zu verbinden, die, bezogen auf die Gebißmitte im Bereich der Schneidezähne, etwa auf gegenüberliegenden Seiten an den Schienen interokklusal angelenkt sind.

Durch die Erfindung ergeben sich eine Reihe von Vorteilen. Durch die interokklusale Anordnung und Lagerung des Stabes weist das erfindungsgemäße Therapiegerät keine von der Oberkieferschiene und der Unterkieferschiene seitlich abstehenden, kantigen und/oder beweglichen Bauteile auf, die beim Tragen des Schnarch-Therapiegerätes, also im Gebrauchszustand, in direkten und dauerhaften Kontakt mit den Wangen kommen. Probleme wie beim Stand der Technik, insbesondere Reizungen des Gewebes an der Innenseite der Wangen, können nicht auftreten. Da die beim Stand der Technik als störend empfundenen Stäbe durch die interokklusale Anordnung im Bereich der Aufbißflächen quasi verschwinden, erhöht sich der Tragekomfort des erfindungsgemäßen Therapiegerätes. Gleichzeitig wird die Akzeptanz beim Benutzer und damit die Tragehäufigkeit verbessert.

Erschlafft beim Einschlafen die Unterkiefermuskulatur, sinkt der Unterkiefer nach unten. Im Gebrauchszustand, also im in den Mund eingesetzten Zustand, bewirkt das Schnarch-Therapiegerät mit dem Absinken des Unterkiefers eine zunehmende Verschiebung des Unterkiefers, wobei die Verschiebung sowohl nach vorne (anterior) als auch nach hinten (posterior) erfolgen kann. Die Verschiebung des Unterkiefers im Gebrauchszustand wird über die Anordnung der Anlenkpunkte des Stabes oder der Stäbe an der Ober- und Unterkieferschiene bestimmt. Ist der Anlenkpunkt der Oberkieferschiene im Gebrauchszustand in geringerem Abstand zu den Schneidezähnen angeordnet als der Anlenkpunkt der Unterkieferschiene, befindet sich also der Anlenkpunkt des Stabes an der Oberkieferschiene bezogen auf die Gebißmitte vor dem Anlenkpunkt des Stabes an der Unterkieferschiene, bewirkt das Schnarch-Therapiegerät eine Verschiebung des Unterkiefers in eine anteriore Lage. Alternativ dazu kann auch vorgesehen sein, daß das Schnarch-Therapiegerät im Gebrauchszustand eine Verschiebung in eine posteriore Lage bewirkt, wenn der Anlenkpunkt der Unterkieferschiene in geringerem Abstand zu den Schneidezähnen als der Anlenkpunkt der Oberkieferschiene angeordnet ist, also wenn der Anlenkpunkt des Stabes an der Oberkieferschiene bezogen auf die Gebißmitte hinter dem Anlenkpunkt des Stabes an der Unterkieferschiene liegt. Diese beiden Ausführungsformen sind hinsichtlich ihrer Funktionalität im wesentlichen gleichwertig. Sowohl durch eine anteriore als auch durch eine posteriore Verschiebung des Unterkiefers wird eine Öffnung der pharyngealen Atemwege erreicht, so daß die Atemluft ungestört, und ohne erschlafftes Gewebe in flatternde Bewegung zu versetzen, in die Luftröhre einund aus dieser wieder austritt. Aufgrund der individuellen anatomischen Verhältnisse des Benutzers kann jedoch in Einzelfällen eine der genannten Ausführungsformen bevorzugt sein.

Von besonderem Vorteil ist es, wenn wenigstens eine Ausnehmung an den einander zugewandten Aufbißflächen zur zumindest im wesentlichen vollständigen Aufnahme des Stabes in der Oberkieferschiene und der Unterkieferschiene, d.h. im Bereich der Aufbißflächen der hinteren Aufbisse, vorgesehen ist. Die Ausnehmung sollte derart ausgebildet sein, daß der Stab in geschlossenem Zustand des Therapiegerätes derart darin aufgenommen ist, daß die Oberkieferschiene und die Unterkieferschiene im Bereich der einander zugewandten Aufbisse zumindest teilweise aufeinander aufliegen, und zwar derart, daß jegliche Störung der Okklusion durch den Stab bzw. die Stäbe ausgeschlossen ist. Die zwischen den Aufbissen angeordneten Stäbe beeinträchtigen die Okklusion zwischen der Oberkieferschiene und der Unterkieferschiene also in keinster Weise. Im Ergebnis ergibt sich damit eine Einbettung des Stabes in den seitlichen bzw. hinteren, einander zugewandten Aufbissen bzw. Aufbißflächen der Schienen. Im geschlossenen Gebrauchszustand des erfindungsgemäßen Schnarch-Therapiegerätes ist damit jede Beeinträchtigung und Einschränkung der Zunge und der Wangenmuskulatur ausgeschlossen.

Um den oder die Stäbe in einfacher Weise an den jeweiligen Schienen befestigen zu können, ist vorgesehen, daß der Stab endseitig Befestigungsabschnitte mit jeweils einer Lageröffnung aufweist. Die Befestigung erfolgt dann derart, daß durch die Lageröffnungen entsprechende Bolzen oder Schrauben hindurchgeschoben werden, die mit der Oberkieferschiene bzw. der Unterkieferschiene verschraubt werden. Für die gegebenenfalls erforderlichen Muttern sind dann entsprechende Ausnehmungen seitlich an der Ober- bzw. Unterkieferschiene vorgesehen.

Alternativ zu der zuvor beschriebenen Ausführungsform ist es grundsätzlich auch möglich, daß der Stab an der Ober- und/oder Unterkieferschiene kugelgelenkig gelagert ist.

Eine besonders bevorzugte Ausgestaltung des erfindungsgemäßen Therapiegerätes sieht vor, daß der in größerem Abstand zu den Schneidezähnen angeordnete Anlenkpunkt des Stabes (hinterer Anlenkpunkt) im oberen Freiraum zwischen zwei benachbarten Zähnen, insbesondere den Prämolaren und den Molaren angeordnet ist. Dadurch kann die zur Lagerung des Stabes erforderliche Höhe des betreffenden Aufbisses verringert werden, wodurch eine weitere Steigerung des Tragekomforts erzielt wird. Die Lage des Anlenkpunktes des Stabes, der im Gebrauchszustand in geringerem Abstand zu den Schneidezähnen angeordnet ist (vorderer Anlenkpunkt), ergibt sich dann automatisch aus der Anordnung des hinteren Anlenkpunktes und der verwendeten Stablänge. Auch der vordere Anlenkpunkt kann in einem oberen Freiraum zwischen zwei benachbarten Zähnen angeordnet sein.

Um eine genau vorgegebene Verstellung des Unterkiefers in Abhängigkeit des Öffnungswinkels zwischen Ober- und Unterkieferschiene im Gebrauchszustand zu erzielen, ist bei einer Ausführungsform vorgesehen, daß die Stäbe in Längsrichtung weder dehnbar noch ausziehbar sind. Bevorzugt werden in diesem Fall Stäbe aus flexiblem Kunststoff verwendet, um eine freie Beweglichkeit des Unterkiefers allerdings mit Ausnahme der Längsrichtung zu ermöglichen. Weiter bevorzugt sind solche flexiblen Kunststoffe, die einen Teil der Bewegungsenergie des Unterkiefers im Gebrauchszustand aufnehmen und dadurch die durch die Bewegung des Unterkiefers auf die Anlenkpunkte einwirkenden Kräfte verringern. Auf diese Weise kann das Schnarch-Therapiegerät trotz hoher mechanischer Belastung während des Tragens vergleichsweise einfach und kleinbauend ausgeführt werden, ohne daß die Lager für den Stab während der Benutzung Schaden nehmen.

Liegen Ober- und Unterkieferschiene des erfindungsgemäßen Geräts im Bereich der seitlichen Aufbisse aufeinander auf (geschlossener Zustand), befindet sich der Stab in einem spitzen Winkel zur seitlichen Aufbißebene. Bei konstanter Länge des Stabes und konstantem Öffnungswinkel zwischen Ober- und Unterkieferschiene ist die durch das erfindungsgemäße Schnarch-Therapiegerät im Gebrauchszustand hervorgerufene Längsverschiebung des Unterkiefers um so größer, je kleiner der Winkel zwischen Stab und Aufbißebene ist. Dadurch können die pharyngealen Atemwege bereits durch einen geringen Öffnungswinkel zwischen Ober- und Unterkiefer geöffnet werden.

Das erfindungsgemäße Schnarch-Therapiegerät erlaubt es grundsätzlich unterschiedliche Stablängen vorzusehen, um das Gerät an die anatomischen Anforderungen des Träger anzupassen. Vorteilhaft in diesem Zusammenhang ist es, daß zu dieser Anpassung an die anatomischen Verhältnisse des Trägers vorgefertigte, standardisierte Stablängen verwendet werden können. Schon mit einer sehr geringen Anzahl unterschiedlicher Stablängen können die unterschiedlichsten anatomischen Verhältnisse berücksichtigt werden.

Statt der zuvor beschriebenen Ausführungsform ist es zur individuellen Anpassung an die Kieferposition eines Patienten grundsätzlich auch möglich, daß der Stab eine Spindel mit einem Bedienelement aufweist und daß der Stab auch im mit der Oberkieferschiene und der Unterkieferschiene verbundenen Zustand durch eine Drehung des Bedienelementes stufenlos längenverstellbar ist. Durch diese Ausgestaltung ergibt sich ein Stab, der nicht nur sehr einfach eine individuelle Anpassung der Kieferschienen ermöglicht, und zwar sogar im eingesetzten Zustand des Therapiegerätes, sondern der auch sehr einfach und damit kostengünstig ist.

Bevorzugt ist es, den Stab bzw. die Spindel derart auszubilden, daß eine Drehung des Bedienelementes in die eine Richtung eine beidseitige Verlängerung und eine Drehung des Bedienelementes in die andere Richtung eine beidseitige Verkürzung des Stabes bewirkt. Durch diese Ausgestaltung wird es ermöglicht, daß schon bei geringen Drehbewegungen größere Verstellwege in Längsrichtung eingestellt werden können. In diesem Zusammenhang weist der Stab ein erstes äußeres Stabelement und ein zweites äußeres Stabelement auf, die jeweils zum Zusammenwirken mit der Spindel vorgesehen sind. Außenseitig weisen die äußeren Stabelemente vorzugsweise Befestigungsabschnitte zur Befestigung an der Oberkieferschiene bzw. der Unterkieferschiene auf. Bei einer besonders einfachen Ausgestaltung sind die Befestigungsabschnitte als Lageröffnungen ausgebildet, wie dies zuvor bereits beschrieben worden ist.

Weiterhin weisen die äußeren Stabelemente vorzugsweise jeweils eine Gewindehülse mit Innengewinde auf, während die Spindel selbst äußere Spindelabschnitte mit korrespondierenden Außengewinden aufweist. Es versteht sich natürlich, daß es grundsätzlich auch möglich ist, daß die äußeren Stabelemente Gewindebolzen aufweisen, während die inneren Spindelabschnitte hülsenförmig mit Innengewinde ausgebildet sind.

Von weiterem Vorteil ist es im übrigen, daß die äußeren Stabelemente baugleich ausgeführt sind und der Stab insgesamt dreiteilig ausgebildet ist, also lediglich die Spindel und die äußeren Stabelemente aufweist. Insgesamt ergibt sich damit eine einfache und kostengünstige Ausgestaltung.

Da sich bei Drehung des Bedienelementes die äußeren Stabelemente relativ zur Spindel in gleicher Weise bewegen, sollte das Bedienelement mittig an der Spindel vorgesehen sein und die Spindelabschnitte die gleiche Länge haben.

Damit es nicht zu einer unbeabsichtigten Längenverstellung des Stabes kommt, sollten die Gewinde im Bereich der Spindel selbsthemmend sein.

Zur Verstellung ist am Bedienelement wenigstens eine Eingriffsöffnung zum Ansetzen eines Werkzeugs vorgesehen. Nach Einsetzen des Werkzeugs in die Eingriffsöffnung wird durch Aufbringen einer Kraft die Gewinde-Selbsthemmung überwunden und die Spindel kann gedreht werden.

Um eine hinreichend große Eingriffsöffnung zur Verfügung zu stellen und damit es nicht zu unnötigen Kanten im Bereich des Stabes kommt, ist das Bedienelement gegenüber den äußeren Spindelabschnitten verdickt und insbesondere mit den Gewindehülsen außenseitig ausgefluchtet.

Wie bereits eingangs erwähnt, ist es im Zusammenhang mit intraoralen Therapiegeräten wichtig, daß sich durch den Gebrauch des Therapiegeräts eine Öffnung der pharyngealen Atemwege ergibt. Um dies zu fördern, ist erfindungsgemäß vorgesehen, daß an der Innenseite wenigstens einer der Kieferschienen, vorzugsweise an der Oberkieferschiene, im Bereich der Schienenmitte wenigstens ein als Perle ausgebildetes Zungenstimulationselement vorgesehen ist. Dieses Element, das im Gebrauchszustand in Richtung der Zunge vom Kieferschienenkörper absteht, führt dazu, daß die Zunge auch im Unterbewußtsein bzw. während des Schlafes an dem Zungenstimulationselement spielt und sich dabei nach vorne orientiert. Hierdurch ergibt sich eine Öffnung der pharyngealen Atemwege, so daß die Atemluft ungestört in die Luftröhre ein- und aus dieser wieder austritt, so daß ein Schnarchen unterbleibt. Es ist darauf hinzuweisen, daß sich die Anordnung des vorstehend genannten Zungenstimulationselements insbesondere im Zusammenhang mit der eingangs genannten Ausführungsform eignet. Allerdings kommt dieser Ausgestaltung auch eigenständige erfinderische Bedeutung zu, also auch bei Kieferschienen, bei denen der Stab oder die Stäbe nicht interokklusal angeordnet sind.

Damit der Patient das Spielen der Zunge am Zungenstimulationselement als angenehm empfindet, ist dieses Element als Perle oder Kugel auszubilden. Eine derartige, zumindest im wesentlichen kantenfreie Ausbildung hat den Vorteil, daß es nicht zu Reizungen an der Zunge kommt. Bevorzugt sollte das Zungenstimulationselement drehbar gelagert sein, was die Stimulation weiter anregt.

Eine einfache Lagerung des Zungenstimulationselements sieht derart aus, daß ein von der betreffenden Kieferschiene abstehender U-förmiger Bügel mit einem Lagerabschnitt vorgesehen ist. Der Bügel ist mit seinen Enden in die betreffende Kieferschiene eingelassen. Das Zungenstimulationselement weist eine Bohrung auf, durch die der Lagerabschnitt hindurchgeführt ist.

Von besonderem Vorteil ist es in diesem Zusammenhang, daß das Zungenstimulationselement auf dem Lagerabschnitt verschiebbar ist. Die Verschiebbarkeit ergibt sich letztlich dadurch, daß der Lagerabschnitt größer ist als das Zungenstimulationselement. Durch die Verschiebbarkeit des Zungenstimulationselements wird der Reiz zur Stimulation weiter erhöht.

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigt:
- Fig. 1: eine Seitenansicht des erfindungsgemäßen Therapiegerätes im geschlossenen Gebrauchszustand,
- Fig. 2: eine Seitenansicht des Therapiegerätes aus Fig. 1 im geöffneten Gebrauchszustand mit in eine anteriore Lage verschobenem Unterkiefer,
- Fig. 3: eine Seitenansicht einer anderen Ausführungsform des erfindungsgemäßen Therapiegerätes im geöffneten Gebrauchszustand mit in eine posteriore Lage verschobenem Unterkiefer,
- Fig. 4: eine schematische Draufsicht auf ein erfindungsgemäßes Therapiegerät und
- Fig. 5: eine teilweise geschnittene Ansicht eines Stabes zur Verwendung bei einem erfindungsgemäßen Therapiegerät.

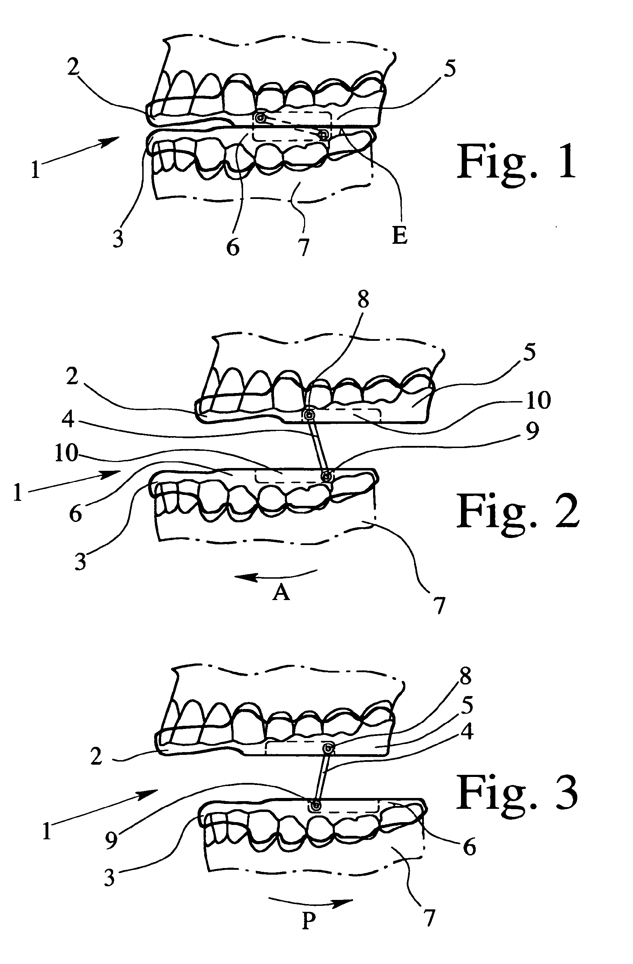

In den Fig. 1 und 2 ist jeweils ein intraorales Therapiegerät 1 dargestellt, bei dem es sich um ein Schnarch-Therapiegerät handelt. Das Therapiegerät 1 weist eine Oberkieferschiene 2 und eine Unterkieferschiene 3 auf. Die beiden Schienen 2, 3 sind vorliegend über zwei (Führungs-)Stäbe 4 miteinander verbunden. Beide Stäbe 4 sind identisch ausgebildet und haben insbesondere die gleiche Länge. Obwohl vorliegend zwei Stäbe 4 vorgesehen sind, darf darauf hingewiesen werden, daß es grundsätzlich auch möglich ist, die beiden Schienen 2, 3 lediglich über einen einzigen Stab miteinander zu verbinden. Jeder der Stäbe 4 ist mit seinem einen Ende an der Oberkieferschiene 2 und mit seinem anderen Ende an der Unterkieferschiene 3 gelenkig gelagert, so daß die Oberkieferschiene 2 und die Unterkieferschiene 3 relativ zueinander verschwenkbar sind, wie dies in Fig. 2 dargestellt ist. Zur Verdeutlichung der Funktion des Therapiegerätes 1 ist dieses in den Fig. 1 bis 3 im Gebrauchszustand, das heißt im in den Mund eingesetzten Zustand, dargestellt. Im übrigen versteht es sich, daß die Oberkieferschiene 2 und die Unterkieferschiene 3 an das Gebiß, das heißt an die Zähne des Benutzers angepaßt sind.

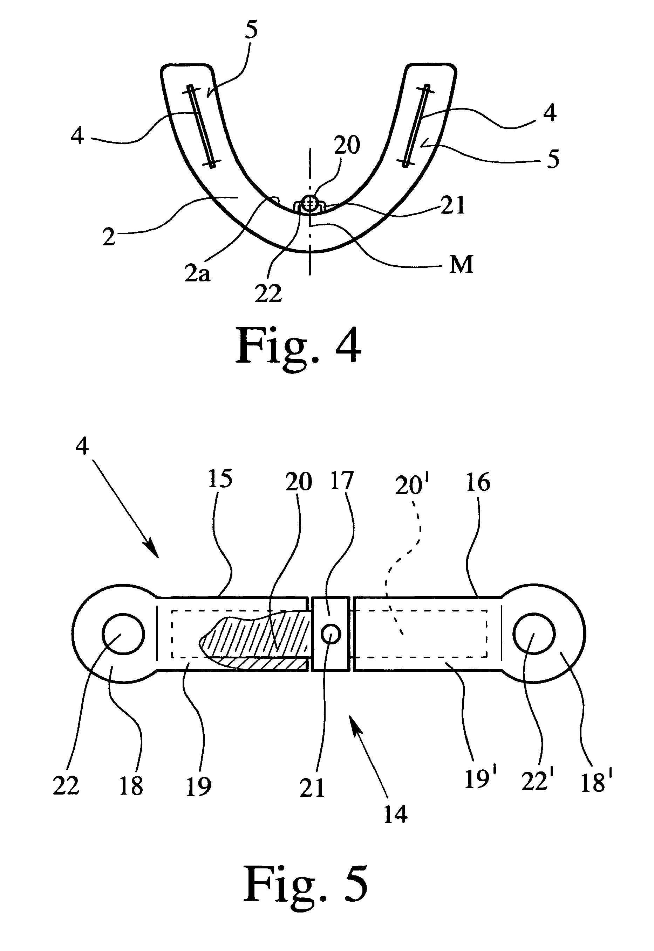

Vorgesehen ist nun, daß jeder der Stäbe 4 interokklusal, also weder vestibulär noch palatinal und lingual angeordnet ist. Jeder der Stäbe 4 ist jeweils einerseits an einem der seitlichen oberen Aufbisse 5 der Oberkieferschiene 2 und andererseits an einem der den jeweiligen oberen Aufbissen 5 gegenüberliegenden, unteren seitlichen Aufbisse 6 der Unterkieferschiene 3 gelenkig gelagert. Damit befinden sich die Lagerungsstellen der Stäbe 4 auf den einander zugewandten Aufbißflächen der Aufbisse 5, 6. Im übrigen sind die beiden Stäbe 4 etwa symmetrisch zur Gebißmitte M an den jeweiligen Aufbissen 5, 6 an beiden Seiten der Schienen 2, 3 gelagert, wie sich dies aus Fig. 4 ergibt.

Bei der in den Fig. 1 und 2 dargestellten Ausführungsform sind die Stäbe 4 derart an der Oberkieferschiene 2 und der Unterkieferschiene 3 angelenkt, daß der Unterkiefer 7 im Gebrauchszustand bei in den Mund eingesetztem Therapiegerät 1 mit dem Öffnen des Mundes nach vorne bewegt wird, wie dies durch den Pfeil A in Fig. 2 dargestellt ist. Zur Realisierung der Bewegung in Pfeilrichtung A ist es bei der in den Fig. 1 und 2 dargestellten Ausführungsform so, daß der Anlenkpunkt 8 des Stabes 4 an der Oberkieferschiene 2 bezogen auf die Gebißmitte M vor dem Anlenkpunkt 9 des Stabes 4 an der Unterkieferschiene 3 liegt. Der obere Anlenkpunkt 8 liegt damit vor dem unteren Anlenkpunkt 9.

Bei der in Fig. 3 dargestellten Ausführungsform ist es hingegen so, daß der Stab 4 derart an der Oberkieferschiene 2 und der Unterkieferschiene 3 angelenkt ist, daß im geöffneten Gebrauchszustand der Unterkiefer 7 nach hinten bewegt wird, wie dies durch den Bewegungspfeil P dargestellt ist. Der Anlenkpunkt 8 des Stabes 4 an der Oberkieferschiene 2 befindet sich dabei - bezogen auf die Gebißmitte M - hinter dem Anlenkpunkt 9 des Stabes 4 an der Unterkieferschiene 3. Unabhängig davon, ob der Unterkiefer 7 in eine anteriore Lage (Fig. 2) oder eine posteriore Lage (Fig. 3) bewegt wird, ist die Verbindung der beiden Schienen 2, 3 über die Stäbe 4 so, daß im Gebrauchszustand eine Öffnung des Mundes gleichzeitig eine Öffnung der pharyngealen Atemwege bewegt.

Um jegliche Störung der Okklusion auszuschließen und gleichzeitig Druckstellen und Einschränkungen der Zungen- und Wangenmuskulatur zu vermeiden, ist in der Oberkieferschiene 2 und der Oberkieferschiene 3 jeweils eine Ausnehmung 10 zur Aufnahme der jeweiligen Stäbe 4 vorgesehen. Die Ausnehmungen 10 sind dabei jeweils im Bereich der Aufbisse 5, 6, also letztlich auf den oberen Aufbißflächen angeordnet. Die Ausbildung und Tiefe der Ausnehmungen 10 ist dabei an den jeweiligen Stab 4 angepaßt. Im geschlossenen Zustand des Therapiegerätes 1, wie er beispielsweise in Fig. 1 dargestellt ist, liegen die Oberkieferschiene 2 und die Unterkieferschiene 3 im Bereich der einander zugewandten Flächen der Aufbisse 5, 6 aufeinander auf, wobei die Stäbe 4 hiervon völlig unbeeinflußt sind, da sie vollständig in die Ausnehmungen 10 eingebettet bzw. darin aufgenommen sind.

Wie sich im übrigen insbesondere aus den Fig. 1 bis 3 ergibt, ist der hintere Anlenkpunkt des Stabes 4 in einem oberen Freiraum zwischen zwei benachbarten Zähnen angeordnet. Auf diese Weise können die seitlichen Aufbisse 5, 6 relativ schmal bzw. mit einer geringen Höhe ausgeführt werden, was den Tragekomfort des Therapiegerätes 1 erhöht.

Die in den Fig. 1 bis 3 dargestellten Stäbe 4 sind im übrigen flexibel und in Längsrichtung nicht dehnbar oder ausziehbar. Grundsätzlich können sie aus jeglichem Material bestehen. Bevorzugt ist allerdings Kunststoff, wobei der Stab 4 eine solche Ausbildung haben sollte, daß seitliche Bewegungen des Unterkiefers aufgrund der Elastizität des Stabes möglich sein sollten. Im übrigen ist es, wie sich dies aus Fig. 1 ergibt, so, daß der Stab 4 im geschlossenen Zustand einen Winkel mit der Aufbißebene E zwischen 5° und 20° aufweist.

In Fig. 5 ist eine Ausführungsform eines Stabes 4 dargestellt, der aus Metall, insbesondere aus Edelstahl besteht und alternativ zu den in den Fig. 1 bis 3 dargestellten Stäben verwendet werden kann. Schon aufgrund seines Materials ist der Stab 4 als solcher nicht dehnbar oder ausziehbar. Der Stab 4 weist eine Spindel 14 mit einem Bedienelement 17 auf und zeichnet sich dadurch aus, daß er auch im mit der Oberkieferschiene 2 und der Unterkieferschiene 3 verbundenen Zustand durch eine Drehbewegung des Bedienelementes 17 stufenlos längenverstellbar ist. Der Stab 4 ist dabei derart ausgebildet, daß eine Drehung des Bedienelementes 17 in die eine Richtung eine beidseitige Verlängerung und eine Drehung des Bedienelementes 17 in die andere Richtung eine beidseitige Verkürzung des Stabes 4 bewirkt. Die Funktion der Spindel 14 ist also zu beiden Seiten hin wirksam. Dabei versteht es sich, daß die diesbezüglichen Gewinde an der Spindel 14 entsprechend auszubilden sind. Im ursprünglichen Einsetzzustand ist die Spindel 14 halb geöffnet, so daß Verstellungen in beiden Richtungen möglich sind.

Im übrigen weist der Stab 4 ein erstes äußeres Stabelement 15 und ein zweites äußeres Stabelement 16 auf, die jeweils mit der Spindel 14 zusammenwirken. An den Stabelementen 15, 16 sind jeweils endseitig Befestigungsabschnitte 18, 18' vorgesehen, die Lageröffnungen 22, 22' aufweisen. Die Befestigungsabschnitte 18, 18' haben eine geringere Breite als die Stabelemente 15, 16, so daß sie in einem entsprechenden Schlitz an der Oberkieferschiene 2 bzw. der Unterkieferschiene 3 eingesetzt und mittels eines Schraubbolzens, der durch die jeweilige Lageröffnung 22, 22' hindurchgeführt wird, fixiert werden können.

Das erste äußere Stabelement 15 und das zweite äußere Stabelement 16 weisen neben den Befestigungsabschnitten 18, 18' jeweils Gewindehülsen 19, 19' mit Innengewinde auf, während die Spindel 14 äußere Spindelabschnitte 20, 20' mit korrespondierenden Außengewinden aufweist. Die Gewinde sind dabei derart ausgebildet, daß sie selbsthemmend sind, so daß eine unbeabsichtigte Längenverstellung des Stabes 4 nicht erfolgen kann.

Wie sich aus Fig. 5 ergibt, sind die Stabelemente 15, 16 baugleich ausgebildet, so daß der Stab 4 mit den beiden Stabelementen 15, 16 und der Spindel 14 insgesamt nur dreiteilig ausgeführt ist. Wie sich im übrigen aus Fig. 5 ergibt, ist das Bedienelement 17 mittig an der Spindel 14 vorgesehen, wobei die Spindelabschnitte 20, 20' die gleiche Länge haben. Am Bedienelement 17 befindet sich vorliegend wenigstens eine Eingriffsöffnung 21 zum Ansetzen eines Werkzeugs. Nach Einsetzen des Werkzeugs und Ausübung einer entsprechenden Kraft wird die Hemmung der Gewinde überwunden und es kann eine Längenverstellung des Stabes 4 erfolgen. Das Bedienelement 17 selbst ist gegenüber den äußeren Spindelabschnitten 20, 20' verdickt und ist außenseitig mit den Gewindehülsen 19, 19' ausgefluchtet.

Aus Fig. 4 ergibt sich, daß an der Innenseite 2a der Oberkieferschiene 2 im Bereich der Schienenmitte bzw. Gebißmitte M ein Zungenstimulationselement 20 vorgesehen ist, das von der Oberkieferschiene 2 absteht. Bei dem Zungenstimulationselement 20 handelt es sich erfindungsgemäß um eine Perle bzw. eine Kugel, die drehbar und gleichzeitig verschiebbar gelagert sein kann. Zu diesem Zweck ist ein U-förmiger Bügel 21 vorgesehen, der in die Oberkieferschiene 2 eingelassen ist. Der Bügel 21 weist einen Lagerabschnitt 22 auf, auf dem sich das Zungenstimulationselement 20 befindet. Um die Drehbarkeit und Verschiebbarkeit des Zungenstimulationselements 20 zu gewährleisten, weist dieses eine im einzelnen nicht dargestellte Durchgangsbohrung auf. Im übrigen ist der Durchmesser der Perle kleiner als die Länge des Lagerabschnitts 22, so daß die Perle entlang des Lagerabschnitts 22 hin und her verschoben und gleichzeitig gedreht werden kann. Der Übergang vom Lagerabschnitt 22 zu den seitlichen Schenkeln des Bügels 21 ist im übrigen jeweils gerundet, um unnötige Reizungen der Zungenspitze zu vermeiden.

## Patentansprüche

1. Intraorales Therapiegerät (1), vorzugsweise Schnarch-Therapiegerät, mit einer Oberkieferschiene (2) und einer Unterkieferschiene (3), wobei die Oberkieferschiene (2) und die Unterkieferschiene (3) über wenigstens einen Stab (4) miteinander verbunden sind und wobei der Stab (4) mit seinem einen Ende an der Oberkieferschiene (2) und seinem anderen Ende an der Unterkieferschiene (3) gelenkig gelagert ist, so daß die Oberkieferschiene (2) und die Unterkieferschiene (3) relativ zueinander verschwenkbar sind, **dadurch gekennzeichnet, daß** an der Innenseite der Oberkieferschiene (2) und/oder an der Unterkieferschiene (3) im Bereich der Schienenmitte (11) wenigstens ein als Perle ausgebildetes Zungenstimulationselement (20) vorgesehen ist, wobei im Gebrauchszustand des Therapiegerätes das Zungenstimulationselement (20) in Richtung der Zunge von einem Schienenkörper der Oberkieferschiene (2) und/oder der Unterkieferschiene (3) derart absteht, daß die Zunge im Unterbewußtsein bzw. während des Schlafes an dem Zungenstimulationselement (20) spielt und sich dabei nach vorne orientiert,

2. Intraorales Therapiegerät nach Anspruch 1, **dadurch gekennzeichnet, daß** das Zungenstimulationselement (20) drehbar und/oder verschiebbar gelagert ist.

3. Intraorales Therapiegerät nach einem der Ansprüche 1 oder 2, **dadurch gekennzeichnet, daß** zur Lagerung des Zungenstimulationselements ein von der Schiene abstehender U-förmiger Bügel (21) mit einem Lagerabschnitt (22) vorgesehen ist.

4. Intraorales Therapiegerät nach einem der vorhergehenden Ansprüche, **dadurch gekennzeichnet, daß** der Stab (4) interokklusal angeordnet ist und einerseits an dem oberen seitlichen Aufbiß (5) der Oberkieferschiene (2) und andererseits an dem dem oberen Aufbiß (5) gegenüberliegenden unteren seitlichen Auf biß (6) der Unterkieferschiene (3) gelenkig gelagert ist.

5. Intraorales Therapiegerät nach einem der vorhergehenden Ansprüche, **dadurch gekennzeichnet, daß** die Oberkieferschiene (2) und/oder LJnterkieferschiene (3) wenigstens eine Ausnehmung (10) zur zumindest im wesentlichen vollständigen Aufnahme des Stabs (4) aufweist.

6. Intraorales Therapiegerät nach einem der vorhergehenden Ansprüche, **dadurch gekennzeichnet, daß** die Ausnehmung (10) im Bereich der seitlichen Aufbisse (5, 6) angeordnet ist.

7. Intraorales Therapiegerät nach einem der vorhergehenden Ansprüche, **dadurch gekennzeichnet, daß** der Stab (4) in geschlossenem Zustand des Therapiegerätes (1) derart in der Ausnehmung (10) der Oberkieferschiene (2) und/oder Unterkieferschiene (3) aufgenommen ist, daß die Oberkieferschiene (2) und die Unterkieferschiene (3) im Bereich der einander zugewandten Flächen der Aufbisse (5, 6) zumindest teilweise aufeinander aufliegen.

8. Intraorales Therapiegerät nach einem der vorhergehenden Ansprüche, **dadurch gekennzeichnet, daß** der hintere Anlenkpunkt des Stabes in einem oberen Freiraum zwischen zwei benachbarten Zähnen, insbesondere zwischen den Prämolaren und den Molaren, angeordnet ist.

## Claims

1. Intra-oral therapeutic device (1), preferably a therapeutic device to prevent snoring, with an upper jaw splint (2) and a lower jaw splint (3), the upper jaw splint (2) and the lower jaw splint (3) being connected to each other via at least one rod (4), and the rod (4) being articulated at one of its ends on the upper jaw splint (2) and at its other end on the lower jaw splint (3), such that the upper jaw splint (2) and the lower jaw splint (3) can pivot relative to each other, **characterized in that** at least one tongue-stimulating element (20) designed as a bead is provided on the inside face of the upper jaw splint (2) and/or on the lower jaw splint (3) in the area of the splint middle (11), and, in the state of use of the therapeutic device, the tongue-stimulating element (20) protrudes away from a splint body of the upper jaw splint (2) and/or of the lower jaw splint (3) in the direction of the tongue, in such a way that, when the wearer is subconscious or asleep, the tongue plays on the tongue-stimulating element (20) and in doing so is oriented forwards.

2. Intra-oral therapeutic device according to Claim 1, **characterized in that** the tongue-stimulating element (20) is mounted rotatably and/or displaceably.

3. Intra-oral therapeutic device according to one of Claims 1 and 2, **characterized in that**, to mount the tongue-stimulating element, a U-shaped bracket (21) is provided which protrudes from the splint and has a bearing section (22).

4. Intra-oral therapeutic device according to one of the preceding claims, **characterized in that** the rod (4) is arranged in an interocclusal position and is articulated at one end on the upper lateral bite (5) of the upper jaw splint (2) and articulated at the other end on the lower lateral bite (6) of the lower jaw splint (3) lying opposite the upper bite (5).

5. Intra-oral therapeutic device according to one of the preceding claims, **characterized in that** the upper jaw splint (2) and/or lower jaw splint (3) has at least one recess (10) for receiving the rod (4) at least almost completely.

6. Intra-oral therapeutic device according to one of the preceding claims, **characterized in that** the recess (10) is arranged in the area of the lateral bites (5, 6).

7. Intra-oral therapeutic device according to one of the preceding claims, **characterized in that**, in the closed state of the therapeutic device (1), the rod (4) is received in the recess (10) of the upper jaw splint (2) and/or lower jaw splint (3) in such a way that the upper jaw splint (2) and the lower jaw splint (3) at least partially bear on each other in the area of the mutually facing surfaces of the bites (5, 6).

8. Intra-oral therapeutic device according to one of the preceding claims, **characterized in that** the rear point of articulation of the rod is arranged in an upper free space between two adjacent teeth, in particular between the premolars and the molars.

## Revendications

1. Dispositif thérapeutique intrabuccal (1), de préférence dispositif thérapeutique reniflard, avec un rail de mâchoire supérieure (2) et un rail de mâchoire inférieure (3), dans lequel le rail de mâchoire supérieure (2) et le rail de mâchoire inférieure (3) sont reliés l'un à l'autre au moyen d'au moins une barrette (4) et dans lequel la barrette (4) est articulée par sa première extrémité au rail de mâchoire supérieure (2) et par son autre extrémité au rail de mâchoire inférieure (3, de telle manière que le rail de mâchoire supérieure (2) et le rail de mâchoire inférieure (3) puissent pivoter l'un par rapport à l'autre, **caractérisé en ce qu'**il est prévu sur la face intérieure du rail de mâchoire supérieure (2) et/ou sur le rail de mâchoire inférieure (3), dans la région du milieu du rail (11), au moins un élément de stimulation de la langue (20) sous la forme d'une perle, dans lequel, lorsque le dispositif thérapeutique est en état d'utilisation, l'élément de stimulation de la langue (20) est saillant en direction de la langue à partir d'un corps de rail du rail de mâchoire supérieure (2) et/ou du rail de mâchoire inférieure (3), de telle manière que la langue joue, dans le subconscient ou pendant le sommeil, sur l'élément de stimulation de la langue (20) et soit ainsi orientée vers l'avant.

2. Dispositif thérapeutique intrabuccal selon la revendication 1, **caractérisé en ce que** l'élément de stimulation de la langue (20) peut tourner et/ou coulisser.

3. Dispositif thérapeutique intrabuccal selon l'une quelconque des revendications 1 ou 2, **caractérisé en ce qu'**il est prévu un étrier en forme de U (21) en saillie sur le rail, avec une partie d'appui (22) pour le montage de l'élément de stimulation de la langue.

4. Dispositif thérapeutique intrabuccal selon l'une quelconque des revendications précédentes, **caractérisé en ce que** la barrette (4) est disposée en position intra-occlusale et est articulée d'une part sur l'appareil orthodontique latéral supérieur (5) du rail de mâchoire supérieure (2) et d'autre part sur l'appareil orthodontique latéral inférieur (6) du rail de mâchoire inférieure (3) qui fait face à l'appareil orthodontique supérieur (5).

5. Dispositif thérapeutique intrabuccal selon l'une quelconque des revendications précédentes, **caractérisé en ce que** le rail de mâchoire supérieure (2) et/ou le rail de mâchoire inférieure (3) présentent au moins une cavité (10) permettant de loger au moins essentiellement entièrement la barrette (4).

6. Dispositif thérapeutique intrabuccal selon l'une quelconque des revendications précédentes, **caractérisé en ce que** la cavité (10) est disposée dans la région des appareils orthodontiques latéraux (5, 6).

7. Dispositif thérapeutique intrabuccal selon l'une quelconque des revendications précédentes, **caractérisé en ce que**, lorsque le dispositif thérapeutique (1) est en position fermée, la barrette (4) est logée dans la cavité (10) du rail de mâchoire supérieure (2) et/ou du rail de mâchoire inférieure (3) de telle manière que le rail de mâchoire supérieure (2) et le rail de mâchoire inférieure (3) s'appliquent au moins partiellement l'un sur l'autre dans la région des faces tournées l'une vers l'autre des appareils orthodontiques (5, 6).

8. Dispositif thérapeutique intrabuccal selon l'une quelconque des revendications précédentes, **caractérisé en ce que** le point d'articulation postérieur de la barrette est disposé dans un espace libre supérieur entre deux dents voisines, en particulier entre les prémolaires et les molaires.
